# EUROPEAN PATENT SPECIFICATION

(11) **EP 1 313 527 B1**
(45) Date of publication and mention of the grant of the patent: **10.01.2007**
(21) Application number: 01968213.7
(22) Date of filing: 28.08.2001
(51) Int. Cl.: A61M 25/00

(54) **MULTI-LUMEN CATHETER AND TIP CONFIGURATIONS FOR USE THEREWITH**
KATHETER MIT MEHREREN LEITUNGEN UND SPITZENKONFIGURATION ZUR VERWENDUNG DAMIT
CATHETER MULTI-LUMIERE ET STRUCTURES DE BOUTS DESTINES A UN TELS CATHETER

(30) Priority: 28.08.2000 US 649680
(43) Date of publication of application: 28.05.2003
(73) Proprietor: C.R. BARD, INC., Murray Hill New Jersey 07974 (US)
(72) Inventor: DIFIORE, Attilio, Taylorsvile, UT 84123 (US); KESTER, Raymond, Lee, Jr., South Jordan, UT 84095 (US)
(74) Representative: Price, Nigel John King
(86) International application number: PCT/US2001/026849
(87) International publication number: WO 2002/018004

(56) References cited:
- EP-A- 0 495 263
- WO-A-97/17102
- CA-A- 1 150 122
- US-A- 5 242 398
- US-A- 5 807 318

## Description

### BACKGROUND OF THE INVENTION

### 1. Field of the Invention

The present invention relates generally to medical devices, and more particularly to an improved multi-lumen catheter.

### 2. Description of Related Art

Multi-lumen catheters are used for a variety of applications where it is necessary to have two or more separate fluid pathways. One such application for a multi-lumen catheter is for use in hemodialysis. During hemodialysis, a dual-lumen catheter can be employed to simultaneously accommodate opposing blood flow. More specifically, one lumen carries blood from a patient to a dialysis machine where it is processed for the removal of toxins, while the opposing lumen returns the purified blood to the patient.

Multi-lumen catheters are well known in the art. Dual lumen catheters, incorporating parallel D-shaped lumen to separate the blood flow and increase the rate of blood flow both into and out of the body, improved upon the use of multiple single lumen catheters. Subsequently, a third circular lumen was introduced to the dual lumen catheters to provide an additional inlet/outlet. Such a catheter can be seen in U.S. Pat. No. 5,797,869 to Martin. In Martin, the third lumen is located in the center of the internal septum, between the two semi-circular lumen. This configuration is intended to give the physician the option of simultaneously injecting a liquid medication during the dialysis procedure, an option not available with a simple dual lumen. However, locating a central lumen between the blood-carrying lumen compromises the reliability of blood flow. Another disadvantage in the catheters of the type described in Martin is that the inlet and outlet openings of the lumen are in close proximity to one another so that toxic blood and purified blood can mix, reducing the efficiency of the process.

A multi-qlumen catheter apparatus according to the preamble of claim 1 is disclosed in EP-A-0 495 263.

### SUMMARY OF THE INVENTION

Accordingly, the present invention provides an improved multi-lumen catheter apparatus according to claim 1 for the simultaneous injection and withdrawal of fluids to and from a patient. The multi-lumen catheter of the present invention is preferably made of polyurethane and is a hollow, cylindrical structure. It includes an outer catheter, generally used for the intake of fluid from the body, and an inner catheter, generally used for injection of fluids into the body. The inner catheter is contained within the outer catheter and extends throughout its length. The hardness of the material used for the inner catheter and outer catheter can be varied, depending on the particular use of the catheter. The material used for the intake lumen should be harder to avoid lumen collapse due to suction, whereas the material used for the outflow lumen should be softer to alleviate trauma of the vein into which the catheter is inserted.

The inner catheter is bisected longitudinally by a septum, which creates two parallel lumens. The shape of each lumen is dependent on the shape of the septum, which can be widely varied. In the case that the inner catheter is split in half, the resulting two lumens are D-shaped. The inner catheter is secured by either the outer catheter's tapered distal end, or a bond, wherein the bond secures the inner catheter to the outer catheter. In a preferred embodiment, the inner catheter extends beyond the distal end of the outer catheter to promote better differentiation between the intake fluid and the outflow fluid. In the case where the multi-lumen catheter is used for hemodialysis, the different lengths of outer and inner catheters help to keep the untreated blood and the purified blood separate. In addition, the two lumens contained within the inner catheter can be of different lengths to maintain fluid separation. The distal end of the two lumens contained within the inner catheter can be varied to improve reliability of fluid flow and avoid vein wall occlusions. For instance, the tips can be beveled, curved, slanted or otherwise altered so that occlusion is less likely. Further, side openings can be cut into either the outer catheter, the inner catheter, or both to improve the reliability of blood flow in the case that one of the lumens becomes occluded. Moreover, a mandrel could be inserted into one of the two lumens within the inner catheter, so that if one of the tubes did become occluded, the mandrel could be removed to provide an open pathway. The lumen could also accompany a guidewire for placement of the catheter.

These and other features and advantages of the present invention will become more apparent to those skilled in the art when taken with reference to the following more detailed description of the preferred embodiments of the invention and the accompanying drawings.

### BRIEF DESCRIPTION OF THE DRAWINGS

Fig. 1 longitudinal sectional view of a multi-lumen catheter.
Fig. 2 is a cross-sectional view of Fig. 1 along the line 2-2.
Fig. 3 is a side view of an alternate embodiment of the multi-lumen catheter.
Fig. 4 is a cross-sectional view taken along line 4-4 of Fig. 3.
Fig. 5 is a side view of an alternate embodiment of the multi-lumen catheter.
Fig. 6 is cross-sectional view of a one configuration for the inner catheter of the multi-lumen catheter.
Fig. 7 is a cross-sectional view of an alternate configuration for the inner catheter of the multi-lumen catheter.
Fig. 8 is a cross-sectional view of another alternate configuration for the inner catheter of the multi-lumen catheter.
Fig. 9 is a side view of an alternate embodiment of the multi-lumen catheter.
Fig. 10 is a side view of the embodiment in Fig. 9 with a bolus tip configuration.
Fig. 11 is a cross-sectional view of the multi-lumen catheter shown in Fig. 10.
Fig. 12 is a side view of a distal end of the multi-lumen catheter shown in Fig. 10, encased in a protective sheath.
Fig. 13 is a side view of the embodiment in Fig. 12 with the lumens exposed.
Fig. 14 is a side view of a proximal end of the embodiment in Fig. 12.
Fig. 15 is a side view of a proximal end of Fig. 13.
Fig. 16 is a side view of an alternate embodiment of the present invention, showing a protective sheath around the outer catheter.

### DETAILED DESCRIPTION OF THE INVENTION

The present invention satisfies the need for improved multi-lumen catheters. More particularly, the present invention provides multi-lumen catheter, a protective sheath and tip configurations that are efficient and effective in reliably transporting fluids to and from a patient, while avoiding fluid mixing and occlusions. In the detailed description that follows, it should be appreciated that like reference numerals are used to describe like elements illustrated in one or more of the figures.

The multi-lumen catheter is a catheter with three or more lumens in which an outer catheter generally surrounds multiple inner lumens. The lumens can be used in any combination for the inflow and outflow of fluids to and from a patient. Further, any one of the lumens can accommodate a mandrel or wire for positioning the multi-lumen catheter. The mandrel or wire can be removed immediately once the catheter is correctly positioned in the patient, or can be left in place, to be removed and used for fluid flow upon the occlusion of one of the other lumens. The multi-lumen catheter can be created by combining an outer catheter with either an inner catheter that has been divided, or with several individual lumens.

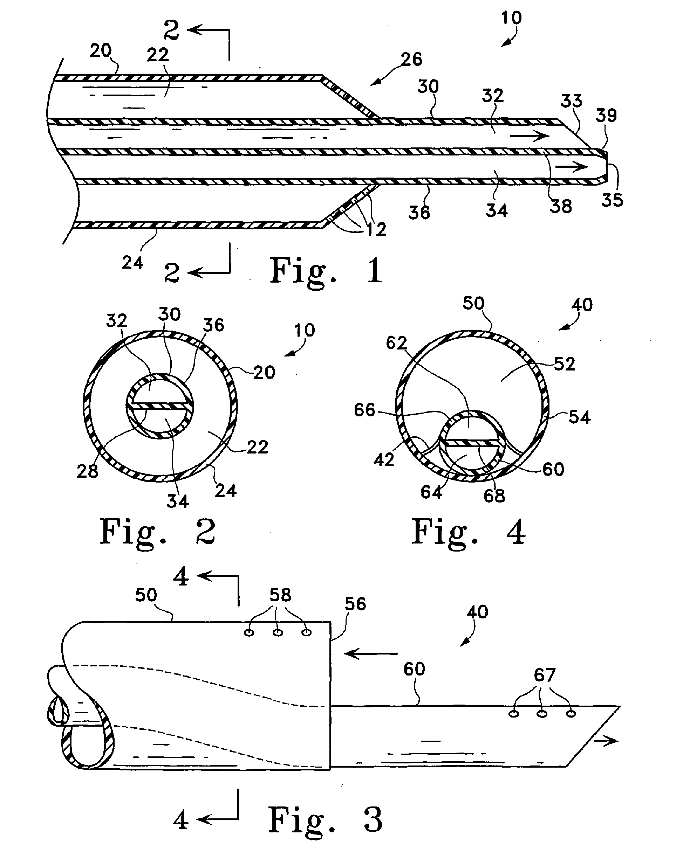

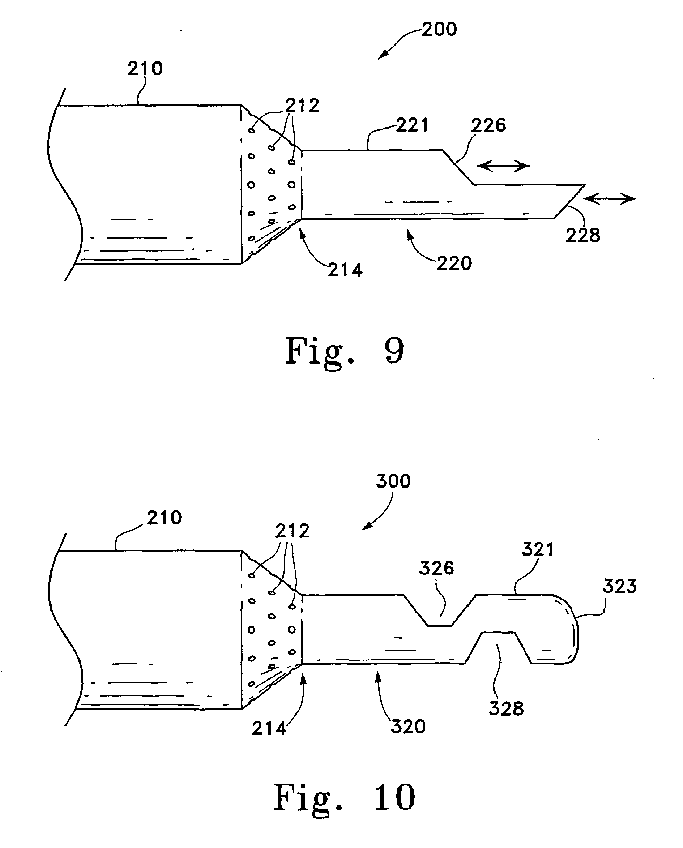

Turning now to Fig. 1, a multi-lumen catheter is illustrated. A longitudinal sectional view of a multi-lumen catheter **10** reveals two tubes with three separate passageways. An outer catheter **20** is a hollow, cylindrically-shaped structure that contains a passageway **22,** which allows the withdrawal of blood for purification through openings **12** located at or near a distal end **26** (see Fig. 9). The outer catheter **20** is disposed circumferentially around an inner catheter **30,** which is also cylindrical, and includes two parallel lumens which are both used for supplying fluid to the patient as indicated by the arrows. In a hemodialysis application, the return fluid is purified blood. Inner catheter **30** includes a first lumen **32** and a second lumen **34,** and has a smaller diameter than the outer catheter **20** so that it can be positioned within the outer catheter **20.** The inner catheter **30** is secured in the multi-lumen catheter **10** by the outer catheter **20,** which tapers at the distal end **26** from its outer diameter to the outer diameter of the inner catheter **30.**

The inner catheter **30** extends beyond the distal end **26** of the outer catheter **20,** terminating at a distal end **39.** At the distal end **39** of the inner catheter **30,** a first outlet **33** of the first lumen **32** is preferably beveled to reduce the chance of vein wall occlusion and other problems associated with blockage of the catheter lumen. A second outlet **35** is preferably blunt, however, to provide a different outlet angle for the fluid. Outlets **33** and **35** can, however, be any configuration known to those skilled in the art. The first and second outlets **33** and **35,** while primarily used for the outflow of cleaned blood to the patient, can also be used for other purposes as well. For example, looking at Fig. 1, the edges near the second outlet **35** are tapered inward to better accommodate a guidewire or mandrel. Fig. 1 also shows the second lumen **34** extending slightly beyond the distal end of first lumen **32.** The two inner lumen of the present invention can end at the same point or be staggered.

As seen in Figs. 1 and 2, the first and second lumens **32** and **34** are separated by a septum **38,** which extends the length of the multi-lumen catheter **10** from a proximal end, where the individual lumens are joined together, to the distal end **39** of the inner catheter **30.** Preferably septum **38** divides the two lumens horizontally into D-shaped cross-sections as shown in Fig. 2, and is preferably made of polyurethane. The wall **24** of the outer catheter **20** and the wall **36** of the inner catheter **30** are also preferably made of polyurethane, but may be made of other recognized materials. The relative hardness of the polyurethane used for the outer wall **24** and the inner wall **36** of the multi-lumen catheter **10** can be varied to achieve a desired result. For example, a harder polyurethane (higher durometer reading) can be used for the outer catheter **20** and a softer polyurethane (lower durometer reading) can be used for the inner catheter **30.** By constructing the multi-lumen catheter in this fashion, the outer catheter **20** has greater structural strength to retain its form under the negative pressure created by the withdrawal of blood from the body, while the inner catheter **30** is softer to prevent the risk of vessel wall erosion. Other variations include using different materials for the outer catheter **20** and inner catheter **30,** or using a soft tip (low durometer reading) for either the distal end **26** of the outer catheter **20** or the distal end **39** of the inner catheter **30.**

Turning now to Fig. 3, an alternate embodiment of the multi-lumen catheter is shown. A side view of a multi-lumen catheter **40** is illustrated, showing an inner catheter **60** extending beyond a distal end **56** of an outer catheter **50.** As in the first embodiment, the outer catheter **50** withdraws fluid, while the inner catheter **60** infuses fluid into the body, as indicated by the arrows. The multi-lumen catheter **40** also has three side openings **58** located near the distal end **56** of the outer catheter **50** as well as three side openings **67** located near the distal end **69** of the inner catheter **60.** These openings can be varied in size and serve the purpose of ensuring the flow of fluids in the event of a whole or partial occlusion of the inner catheter **60** or the outer catheter **50,** as well as improving mixing. The distal end **69** of the inner catheter **60** is beveled to reduce the chance of occlusion from the vein wall. Tapering can be accomplished thermally, using heat and dies, or can be produced by molding. Fig. 4 shows a cross-sectional view of Fig. 3 along line 4-4. The multi-lumen catheter **40** is shown with inner catheter **60** disposed at a point abutting the wall **54** of the outer catheter **50.** The inner catheter **60** is maintained in this position by a weld **42** at the distal end **56** of the outer catheter **50,** which joins the wall **66** of the inner catheter **60** with the wall **54** of the outer catheter **50** on the interior of the outer catheter **50.** The inner catheter **60** is divided horizontally along its entire length by a septum **68,** creating two D-shaped passageways, a first lumen **62** and a second lumen **64.**

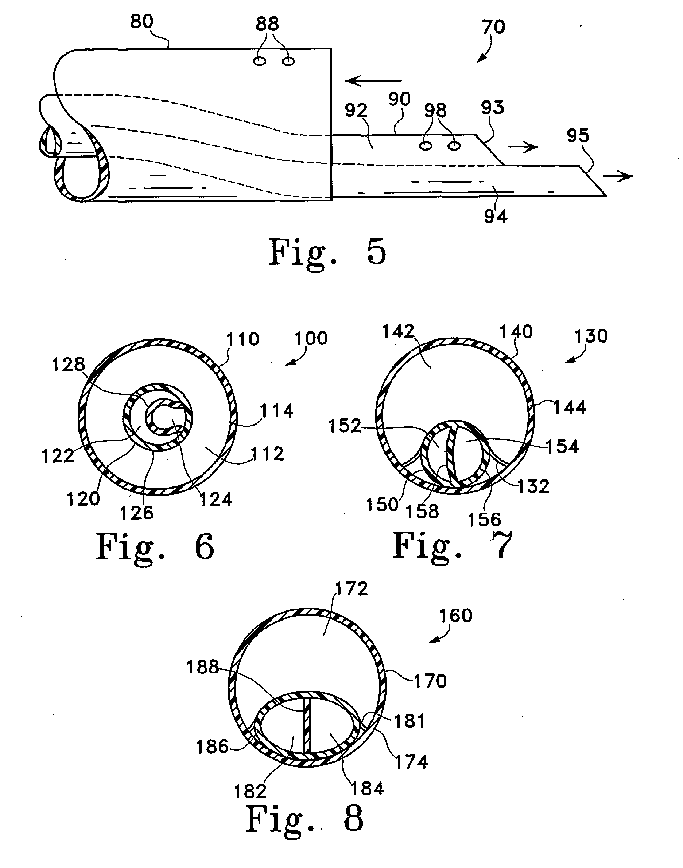

As shown by the dotted lines in Fig. 3, the portion of the inner catheter **60,** which remains within the outer catheter **50** preferably has a sharp slope to a concentric position within the outer catheter **50.** This is best accomplished, as shown in Figs. 3 and 5, by stabilizing the inner catheter **60** in a central position within the outer catheter **50.** The sharp slope created by such a configuration decreases the likelihood of clotting problems that would occur with a more gradual slope if, for example, the inner catheter were allowed to float free within the outer catheter **50.** It should be appreciated that the inner catheter **60** may be bonded to the outer catheter **50** at other locations and in other ways. For instance, a weld could be placed on the exterior of both the outer catheter **50** and the inner catheter **60** at the distal end **56** of the outer catheter **50.** Alternatively, the inner catheter **60** could be glued to the outer catheter **50** at any point along the length of the outer catheter **50.**

Fig. 5 illustrates a side perspective view of another alternate embodiment. A multi-lumen catheter **70** has an outer catheter **80** and an inner catheter **90.** As in Fig. 3, both the outer catheter **80** and the inner catheter **90** have side openings **88** and **98,** respectively, for improved reliability of lumen patency and mixing of infused fluid. It should be appreciated, however, that either the outer catheter or inner catheter or both could be constructed without openings **88** and **98.** The inner catheter **90,** as depicted by the dotted line, contains two opposing parallel lumens, a first lumen **92** and a second lumen **94.** The first lumen **92** and the second lumen **94** terminate in beveled ends **93** and **95** respectively. The first lumen **92** is longitudinally shorter than the second lumen **94,** allowing the multi-lumen catheter **70** to have three stages of flow. This configuration provides more options for mixing and for overcoming occlusion problems that may arise. In an alternate embodiment, the multi-lumen catheter **70** includes a tip portion of the second lumen **94** that is more flexible to reduce the possibility of occlusion, and to lessen the trauma on the vein wall that would occur with a less flexible, harder tip.

Figs. 6-8 show cross-sections of alternate configurations of the inner catheter of the present invention. A multi-lumen catheter **100** of Fig. 6 includes an outer catheter **110** and an inner catheter **120.** The outer catheter **110** is a hollow cylindrical structure with a wall **114** and an inner passageway **112.** The inner catheter **120** contains a first lumen **122** and a second lumen **124,** divided by a C-shaped septum **128.** A wall **126** of the inner catheter **120** isolates the liquid flowing through the inner catheter **120** from the liquid flowing through the outer catheter **110.** The inner catheter **120** is not bonded to the outer catheter **110** in this configuration, and instead is secured in the catheter **100** by a tapered end of the outer catheter **110,** as in Fig. 1. Fig. 7 shows a multi-lumen catheter **130,** wherein a wall **156** of an inner catheter **150** is bonded to a wall **144** of an outer catheter **140** by a bond **132.** The inner catheter **150** is separated into a first lumen **152** and a second lumen **154** by a septum **158,** which bisects the inner catheter **150** essentially vertically, but whose cross-section is bowed in one direction. Thus, a first lumen **152** is crescent shaped, as the bowed shape of the septum **158** bows into the lumen **152.** The second lumen **154,** as a result, has a cross-sectional area slightly greater than that of a D-shaped cross section as shown in Fig. 4. Making the D-shape of the lumens unequal in size by altering the septa **128** and **158** allows issues of structural integrity and unequal flow requirements to be addressed. It should be appreciated by those skilled in the art that, depending on the functional need of the inner catheter of the present invention, the shape of the septum can be alternately configured. For example, the inner catheter could be divided in such a way that three lumen result from the division. Finally, Fig. 8 shows yet another possible configuration for the cross-section of the inner catheter. A multi-lumen catheter **160** has an inner catheter **180,** which is oval as opposed to the circular examples shown above. The oval wall **186** separates the inner catheter **180** from the fluid flow of the outer catheter **170** and is bonded to the outer catheter 170 by a bond **181.** The inner catheter **180** is vertically bisected by a septum **188,** producing D-shaped lumens **182** and **184.**

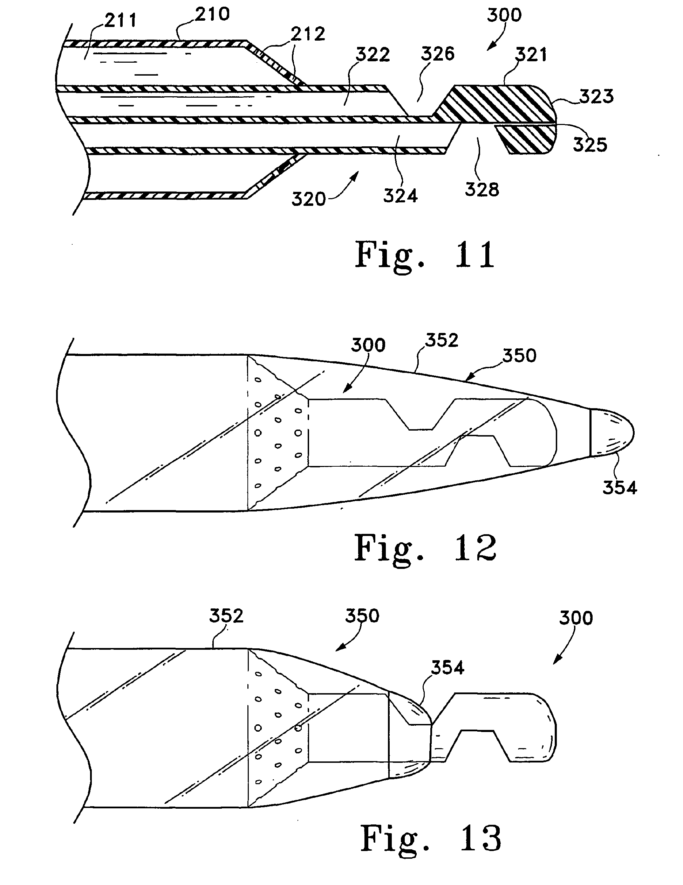

Figs. 9-11 illustrate a preferred embodiment of the multi-lumen catheter. In this embodiment, the inner catheter is held in a central position at the distal end by the narrowing configuration of the outer catheter. Referring to Fig. 9, a multi-lumen catheter **200** includes an outer catheter **210** and an inner catheter **220.** The outer catheter **210** has a distal end **214** that narrows to a smaller diameter to accommodate the inner catheter **220.** The smaller diameter of the distal end **214** of the outer catheter **210** permits some movement of the inner catheter **220** even though the fit between the outer catheter **210** and the inner catheter **220** at the distal end **214** is sufficiently close to prevent any unwanted drifting of the inner catheter **220.** When desired, however, the inner catheter **220** can be moved in a proximal or distal direction to optimize the flow of blood. The distal end **214** of the outer catheter **210** contains side openings **212** which can be varied in number and size and serve the purpose of allowing the withdrawal of blood to a passageway **211** (see Fig. 11) of the outer catheter **210.** The inner catheter **220** is divided into a first lumen and a second lumen, and has a tip **221.** The first lumen has a beveled opening **226** that is slanted toward the outer catheter **210** as shown in Fig. 9 while the second lumen has a beveled opening **228** that is slanted away from the outer catheter **210.** By orienting the bevels in opposite directions, greater separation of blood can be accomplished. As indicated by the arrows, both lumen can support the inflow or outflow of blood.

Fig. 10 illustrates a multi-lumen catheter **300** that includes an outer catheter **210** and an inner catheter **320.** The outer catheter **210,** described above, accommodates the inner catheter **320,** which includes a bolus tip **321** as well as a first lumen **322** and a second lumen **324** (see Fig. 11). The bolus tip **321** is unlike any of the other tips described above in that the openings to the first and second lumens are not located at the distal end of the inner catheter **320.** Instead, the first lumen **322** has an opening **326** on one side of the bolus tip **321,** wherein a section of the bolus tip **321** has been removed, extending down within the first lumen **322,** but short of the second lumen **324** as shown in Fig. 11. Similarly, the second lumen **324** has an opening **328** preferably approximately 180° radially from the opening **326,** wherein the opening **328** is horizontally located distal of the opening **326.** The bolus tip **321** also includes a rounded nose 323 to prevent unnecessary trauma to the patient's vessel in which it is placed. Variations on the bolus tip **321** can be found in U.S. Pat. No. 6,786,884.

Referring to the cross-sectional view of multi-lumen catheter **300** in Fig. 11, the tip **321** preferably includes lumen **325** which is added to the tip **321** to accommodate a guidewire. Lumen **325** is shown connecting the second lumen **324** to the end of the nose **323,** although it will be appreciated that the guidewire lumen **325** could just as easily be connecting the first lumen **322** to the end of the nose **323.**

Figs. 12 and 13 illustrate an embodiment of the present invention. The distal end of the multi-lumen catheter 300 is shown encased in a protective sheath **350.** The sheath **350,** at its distal end, has a main portion **352,** which can be tapered and friction fit to the outside of the multi-lumen catheter **300** to snuggly fit around all portions of both the outer catheter **210** and the inner catheter **320,** and a self-sealing cap portion **354.** Fig. 12 illustrates the catheter **300** when sealed by the sheath **350.** The catheter **300** can be introduced to a bodily vessel in this configuration to keep any unwanted fluids or bacteria from entering the lumen. Moreover, the sheath **350** protects the catheter **300** while it is within the bodily vessel and lowers the probability of clotting because it enables the physician to withdraw the inner catheter **320** from the blood stream. Furthermore, if it becomes necessary to flush the lumens of the inner catheter **320,** the sheath **350** may permit the physician to use a fluid other than heparin and still maintain patency of the device. Any reduction in the amount of heparin used significantly reduces costs. Another benefit imparted by the use of the protective sheath **350** is that the inner catheter **320** may have a tendency to rest against a vessel wall, depending on the method of placement and the final resting position of the device. By withdrawing the inner catheter **320** within the protective sheath **350,** fibrin sheath formation is restricted to the outer catheter **210.** In fact, the sliding action of the inner catheter **320** into and out of the outer catheter **210** may actually remove any fibrin sheath formation on the outer catheter **210,** or at least function to reduce the clotting effects associated therewith.

Fig. 13 shows the catheter **300** in an open configuration, wherein the sheath **350** seals only a portion of the catheter **300.** In this particular case, the flow of fluid into the arterial lumen **322** (see Fig. 11) is partially restricted by the cap portion **354** of the sheath **350** that blocks a portion of the opening **326** (see Fig. 11). Thus, the sheath **350,** in addition to providing a sterile environment for the catheter **300,** can also be used to regulate the flow of fluids. Of course, due to the highly pliable nature of the cap portion **354,** movement is also possible in the opposite direction, in the case that one wants to re-seal the multi-lumen catheter **300.** Because the catheter **300** can be sealed and re-sealed by the sheath **350,** catheter patency is increased. Further, as mentioned above, the lumens do not need to be heparinized and flushed if they are sealed within the sheath **350.** On the other hand, if heparin flushing is required, the sealing aspect of the sheath **350** makes it useful as a heparin lock to prevent clotting and to prevent the toxic release of heparin into the blood.

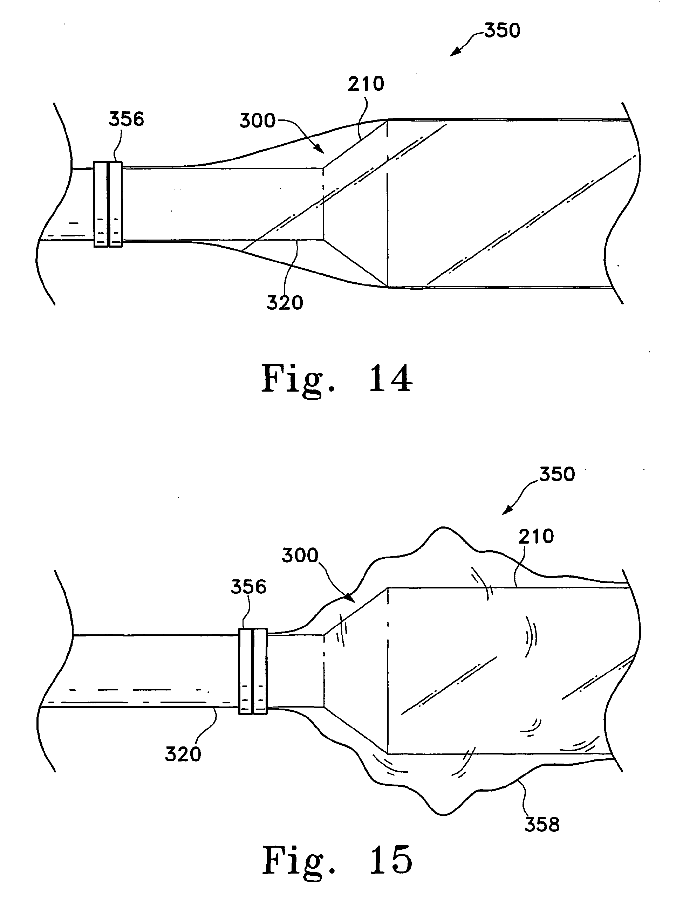

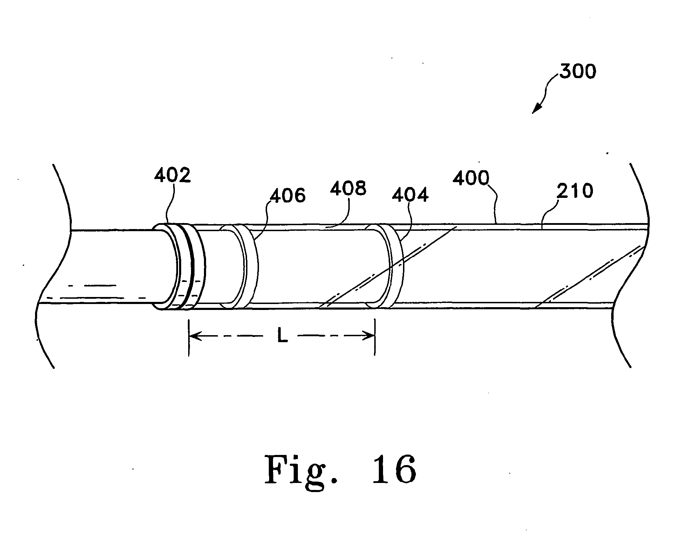

Figs. 14 and 15 show a view of the proximal end of a multi-lumen catheter of the present invention with the sheath **350** over the top of an outer catheter. While not shown in these illustrations, one of the primary benefits of the sheath **350** with the multi-lumen catheters of the present invention is the fact that the sheath **350** can be directly attached to the body. As is well known to those of skill in the art, catheters that are attached to a patient's body for an extended period become fixed in place due to growth and infiltration of the body's cells into the outer wall of the catheter. When the catheter is to be moved in a proximal or distal direction, the portion of the catheter that has become fixed to the body is torn from its bond, potentially causing irritation and discomfort to the patient and acting as a site of potential infection. The use of the sheath 350 overcomes this potential problem because the sheath is composed of soft polyurethane or silicon at the point of attachment and does not move with respect to the body when the catheter is displaced in a proximal or distal direction.

As can be seen in Fig. 14, the sheath **350** is sealingly attached to the inner catheter **320** with a valved O-ring **356.** It should be appreciated, however, that the sheath could be attached to the catheter **300** in a variety of different ways. The proximal view of Fig. 14 corresponds to Fig. 12, which shows the catheter **300** in a closed configuration, sealed from outside fluids and possible contaminants. Fig. 15 illustrates what occurs when it is desired to open the catheter **300** to bodily fluids. The inner catheter **320** is pushed from its proximal end, through the self-sealing cap portion **354** until the desired length of the inner catheter **320** is exposed. The O-ring **356** is attached to the inner catheter **320** at its proximal end, creating a sterile environment for the enclosed surface of the catheter **300** when the catheter is manipulated in a proximal and distal direction. When the catheter **300** is exposed to the bodily vessel by pushing the inner catheter **320** in a distal direction so that the nose **323** of the inner catheter **320** extends outside the sheath **350,** as can be seen in corresponding Fig. 13, the sterility of catheter **300** is maintained because of the sealed attachment by ring **356.** Referring again to Fig. 15, the collapsible portion **358** of the sheath **350** that is located outside of the body and proximal to the attachment point of the body, can be made of a softer polyurethane or silicone than the main portion **352,** which can be composed of a higher durometer material. As the inner catheter **320** is advanced in a distal direction, the collapsible portion **358** bunches, but maintains a sterile lock with respect to the inner catheter **320** and the outer catheter **210.** This configuration allows the movement of the distal end of catheter **300** in and out of the self-sealing sheath **350,** permitting control of fluid flow as well as providing sterility and increased catheter patency.

Fig. 16 illustrates an alternate embodiment of the present invention, showing another means for utilizing a protective sheath. In this embodiment, the multi-lumen catheter **300** is encased in a protective sheath **400,** wherein a longitudinal length **L** of the sheath **400** is filled with an antibacterial agent. The length **L** is the distance measured from the valved O-ring **402,** which seals the sheath **400** at its proximal end, to a stay **404** located distally of the O-ring **402,** which prevents distal movement of the outer catheter **210** as will be explained in more detail below. The length **L** can be any suitable length based on the particular application. Valved O-ring **402** and stay **404** provide a seal around outer catheter **210** over length **L,** but are not attached to the outer catheter **210** such that the catheter **210** can slide in a proximal or distal direction. However, a damper **406** which is attached to the outer catheter **210** allows travel only over length **L.** For example, when it is desired to move the outer catheter **210** in either a distal or proximal direction, the stay **404** will prevent the damper **406** from traveling distally and the O-ring **402** will prevent the damper **406** from traveling proximally, thus limiting the respective movement of the outer catheter **210** to which the damper **406** is attached.

Between the O-ring **402** and the stay **404,** a sterile cylindrical region **408** is created around the outer catheter **210.** Antibacterial agents such as silver-sulfadiazine can be positioned within the region **408** to continually sterilize the outer catheter **210.** To this end, the damper **406** can be made of a porous material such as Dacron or a solid material such as neoprene with holes created therein, allowing the passage of fluid through the damper **406** that will permit the antibacterial agent to sterilize all parts of the catheter within the region **408.**

Many alterations and modifications may be made by those having ordinary skill in the art without departing from the scope of the present invention. For example, a multi-lumen catheter has been illustrated with one outer lumen and one inner lumen, separated by a septum to create two smaller inner lumens. It should be apparent, however, that the inventive concepts described above would be equally applicable to a multi-lumen catheter with a larger number of lumen, such as an outer lumen bisected by a septum, each sub-lumen containing an inner lumen, such inner lumen further divided as described above, for a total of six lumen.

## Claims

1. A multi-lumen catheter apparatus for use in the simultaneous injection of fluids and aspiration of fluids comprising:
a hollow elongate outer catheter (20) having an outer wall;
an elongate inner catheter (30) disposed within said outer catheter, defining an annular region (22) therebetween, said inner catheter including an enclosing wall (36), a septum (38) and a tip, wherein said septum extends within said enclosing wall of said inner catheter, defining a first (32) and second (34) lumen therein; **characterized by**
a protective sheath (350, 400) extendable over the entire length and around all portions of said inner and outer catheters while said apparatus is within a bodily vessel.

2. The multi-lumen catheter apparatus of Claim 1, wherein said protective sheath includes a self-sealing cap at a distal end thereof that permits the passage of the inner catheter therethrough.

3. The multi-lumen catheter apparatus of Claim 1, wherein said protective sheath includes a sealed region at a proximal end of said apparatus and means for limiting the movement of said apparatus through said region.

4. The multi-lumen catheter apparatus of Claim 3, wherein said sealed region contains an antibacterial agent.

5. The multi-lumen catheter apparatus of Claim 1, wherein said tip of said inner catheter extends longitudinally beyond a distal end of said outer catheter.

6. The multi-lumen catheter apparatus of Claim 1, wherein said septum extends from a proximal end of said inner catheter to said tip.

7. The multi-lumen catheter apparatus of Claim 1, wherein said outer catheter tapers from a first diameter to a smaller second diameter at a distal end thereof.

8. The multi-lumen catheter apparatus of Claim 7, wherein a distal end of said outer catheter abuts an outer surface of said inner catheter to substantially center said inner catheter within said outer catheter.

9. The multi-lumen catheter apparatus of Claim 1, wherein said tip is formed at an angle, and wherein the enclosing wall on a first side of said inner catheter extends longitudinally beyond the enclosing wall on a second side of said inner catheter directly opposite said first side, forming a bevel.

10. The multi-lumen catheter apparatus of Claim 1, wherein said tip is angled from said enclosing wall to said septum on one side of said inner catheter, forming a bevel.

11. The multi-lumen catheter apparatus of Claim 1, wherein said second lumen of said inner catheter extends longitudinally beyond said first lumen.

12. The multi-lumen catheter apparatus of Claim 1, wherein said tip has a first cavity and a second cavity, and wherein said first lumen is in fluid communication with said first cavity and said second lumen is in fluid communication with said second cavity.

13. The multi-lumen catheter apparatus of Claim 12, further comprising a guidewire lumen, wherein said guidewire lumen extends from said first cavity through said end of said tip.

14. The multi-lumen catheter apparatus of Claim 12, further comprising a guidewire lumen, wherein said guidewire lumen extends from said second cavity through said end of said tip.

15. The multi-lumen catheter apparatus of Claim 1, wherein an outer surface of said inner catheter is bonded to said outer catheter near a distal end of said outer catheter.

16. The multi-lumen catheter apparatus of Claim 1, wherein said outer wall further comprises a plurality of openings located near a distal end of said outer catheter, and wherein said annular region is in fluid communication with said openings.

17. The multi-lumen catheter apparatus of Claim 1, wherein said enclosing wall further comprises a plurality of openings located near said tip of said inner catheter wherein said first and second lumens are in fluid communication with said openings.

18. The multi-lumen catheter apparatus of Claim 1, wherein a cross-section of said enclosing wall of said inner catheter is circular.

19. The multi-lumen catheter apparatus of Claim 1, wherein a cross-section of said enclosing wall of said inner catheter is oval.

20. The multi-lumen catheter apparatus of Claim 1, wherein said septum bisects said inner catheter, and wherein a cross-sectional area of both said first and second lumens are D-shaped.

21. The multi-lumen catheter apparatus of Claim 1, wherein said septum is curved.

## Patentansprüche

1. Katheter mit mehreren Leitungen zur Verwendung zur gleichzeitigen Injektion von Flüssigkeiten und Aspiration von Flüssigkeiten, umfassend:
einen hohlen, länglichen Außenkatheter (20) mit einer Außenwand,
einen länglichen Innenkatheter (30) im Innern des Außenkatheters, wodurch ein dazwischen liegender Ringbereich (22) definiert wird, wobei der Innenkatheter eine umschließende Wand (36), ein Septum (38) und eine Spitze beinhaltet, wobei das Septum sich in der umschließenden Wand des Innenkatheters erstreckt, wodurch darin ein erstes (32) und ein zweites Lumen (34) definiert wird,
**gekennzeichnet durch**
eine Schutzhülse (350,400), die über die gesamte Länge und um alle Teile des Innen- und Außenkatheters ausdehnbar ist, während das Gerät sich in einem Körpergefäß befindet.

2. Katheter mit mehreren Leitungen gemäß Anspruch 1, wobei die Schutzhülse eine selbstverschließende Kappe an einem distalen Ende desselben beinhaltet, die den Durchtritt des Innenkatheters durch die Schutzhülse erlaubt.

3. Katheter mit mehreren Leitungen gemäß Anspruch 1, wobei die Schutzhülse einen verschlossenen Bereich an einem proximalen Ende des Katheters sowie Mittel zur Einschränkung der Bewegungsfähigkeit des Katheters durch diesen Bereich beinhaltet.

4. Katheter mit mehreren Leitungen gemäß Anspruch 3, wobei der verschlossene Bereich einen antibakteriellen Wirkstoff enthält.

5. Katheter mit mehreren Leitungen gemäß Anspruch 1, wobei die Spitze des Innenkatheters sich in Längsrichtung über das distale Ende des Außenkatheters hinaus erstreckt.

6. Katheter mit mehreren Leitungen gemäß Anspruch 1, wobei das Septum sich von einem proximalen Endes des Innenkatheters bis zu der Spitze erstreckt.

7. Katheter mit mehreren Leitungen gemäß Anspruch 1, wobei der Außenkatheter sich an einem distalen Ende desselben von einem ersten Durchmesser zu einem kleineren zweiten Durchmesser verjüngt.

8. Katheter mit mehreren Leitungen gemäß Anspruch 7, wobei der Außenkatheter mit einem distalen Ende an einer Außenfläche des Innenkatheters anliegt, so dass der Innenkatheter im Wesentlichen im Inneren des Außenkatheters zentriert wird.

9. Katheter mit mehreren Leitungen gemäß Anspruch 1, wobei die Spitze in einem Winkel geformt ist und wobei die umschließende Wand auf einer ersten Seite des Innenkatheters sich in Längsrichtung über die umschließende Wand auf einer zweiten Seite des Innenkatheters, die sich direkt gegenüber der ersten Seite befindet, hinaus erstreckt und auf diese Weise eine Schrägkante bildet.

10. Katheter mit mehreren Leitungen gemäß Anspruch 1, wobei die Spitze sich in einem Winkel von der umschließenden Wand zu dem Septum auf einer Seite des Innenkatheters erstreckt und auf diese Weise eine Schrägkante bildet.

11. Katheter mit mehreren Leitungen gemäß Anspruch 1, wobei das zweite Lumen des Innenkatheters sich in Längsrichtung über das erste Lumen hinaus erstreckt.

12. Katheter mit mehreren Leitungen gemäß Anspruch 1, wobei die Spitze eine erste Höhlung und eine zweite Höhlung besitzt und wobei das erste Lumen sich im Flüssigkeitskontakt mit der ersten Höhlung und das zweite Lumen sich im Flüssigkeitskontakt mit der zweiten Höhlung befindet.

13. Katheter mit mehreren Leitungen gemäß Anspruch 12, der darüber hinaus ein Führungsdrahtlumen umfasst, wobei das Führungsdrahtlumen sich von der ersten Höhlung durch das Ende der Spitze erstreckt.

14. Katheter mit mehreren Leitungen gemäß Anspruch 12, der darüber hinaus ein Führungsdrahtlumen umfasst, wobei das Führungsdrahtlumen sich von der zweiten Höhlung durch das Ende der Spitze erstreckt.

15. Katheter mit mehreren Leitungen gemäß Anspruch 1, wobei eine Außenfläche des Innenkatheters in der Nähe eines distalen Endes des Außenkatheters mit dem Außenkatheter verbunden ist.

16. Katheter mit mehreren Leitungen gemäß Anspruch 1, wobei die Außenwand darüber hinaus eine Mehrzahl von Öffnungen umfasst, die sich in der Nähe eines distalen Endes des Außenkatheters befinden, und wobei der Ringbereich sich in Flüssigkeitskontakt mit den Öffnungen befindet.

17. Katheter mit mehreren Leitungen gemäß Anspruch 1, wobei die umschließende Wand darüber hinaus eine Vielzahl von Öffnungen umfasst, die sich in der Nähe der Spitze des Innenkatheters befinden, wobei das erste und das zweite Lumen sich im Flüssigkeitskontakt mit den Öffnungen befinden.

18. Katheter mit mehreren Leitungen gemäß Anspruch 1, wobei ein Querschnitt der umschließenden Wand des Innenkatheters kreisförmig ist.

19. Katheter mit mehreren Leitungen gemäß Anspruch 1, wobei ein Querschnitt der umschließenden Wand des Innenkatheters oval ist.

20. Katheter mit mehreren Leitungen gemäß Anspruch 1, wobei das Septum den Innenkatheter schneidet und wobei ein Querschnittsbereich sowohl des ersten als auch des zweiten Lumens D-förmig ist.

21. Katheter mit mehreren Leitungen gemäß Anspruch 1, wobei das Septum gebogen ist.

## Revendications

1. Dispositif de cathéter à conduits multiples destiné à être utilisé pour l'injection de fluides et l'aspiration de fluides simultanées, comprenant :
un cathéter extérieur allongé creux (20) comportant une paroi extérieure ;
un cathéter intérieur allongé (30) disposé à l'intérieur dudit cathéter extérieur, définissant une région annulaire (22) entre ceux-ci, ledit cathéter intérieur comprenant une paroi de confinement (36), une cloison (38) et un bout, ladite cloison s'étendant à l'intérieur de ladite paroi de confinement dudit cathéter intérieur, et définissant un premier (32) et un deuxième (34) conduits à l'intérieur de celui-ci ; **caractérisé par** :
une gaine de protection (350, 400) pouvant s'étendre sur la totalité de la longueur et autour de toutes les parties desdits cathéters intérieur et extérieur tandis que ledit dispositif se trouve à l'intérieur d'un vaisseau corporel.

2. Dispositif de cathéter à conduits multiples selon la revendication 1, dans lequel ladite gaine de protection comprend un capuchon auto-scellant à une extrémité distale de celui-ci, qui permet le passage du cathéter intérieur à travers celui-ci.

3. Dispositif de cathéter à conduits multiples selon la revendication 1, dans lequel ladite gaine de protection comprend une région hermétiquement scellée à une extrémité proximale dudit dispositif et des moyens pour limiter le déplacement dudit dispositif à travers ladite région.

4. Dispositif de cathéter à conduits multiples selon la revendication 3, dans lequel ladite région hermétiquement scellée contient un agent anti-bactérien.

5. Dispositif de cathéter à conduits multiples selon la revendication 1, dans lequel ledit bout dudit cathéter intérieur s'étend longitudinalement au-delà d'une extrémité distale dudit cathéter extérieur.

6. Dispositif de cathéter à conduits multiples selon la revendication 1, dans lequel ladite cloison s'étend d'une extrémité proximale dudit cathéter intérieur audit bout.

7. Dispositif de cathéter à conduits multiples selon la revendication 1, dans lequel ledit cathéter extérieur s'effile d'un premier diamètre à un deuxième diamètre plus petit à une extrémité distale de celui-ci.

8. Dispositif de cathéter à conduits multiples selon la revendication 7, dans lequel une extrémité distale dudit cathéter extérieur est en butée contre une surface extérieure dudit cathéter intérieur de façon à centrer sensiblement ledit cathéter intérieur à l'intérieur dudit cathéter extérieur.

9. Dispositif de cathéter à conduits multiples selon la revendication 1, dans lequel ledit bout est formé avec un certain angle, et dans lequel la paroi de confinement sur un premier côté dudit cathéter intérieur s'étend longitudinalement au-delà de la paroi de confinement sur un deuxième côté dudit cathéter intérieur directement opposé audit premier côté, formant un biseau.

10. Dispositif de cathéter à conduits multiples selon la revendication 1, dans lequel ledit bout présente un certain angle depuis ladite paroi de confinement à ladite cloison sur un côté dudit cathéter intérieur, formant un biseau.

11. Dispositif de cathéter à conduits multiples selon la revendication 1, dans lequel ledit deuxième conduit dudit cathéter intérieur s'étend longitudinalement au-delà dudit premier conduit.

12. Dispositif de cathéter à conduits multiples selon la revendication 1, dans lequel ledit bout comporte une première cavité et une deuxième cavité, et dans lequel ledit premier conduit est en communication de fluide avec ladite première cavité et ledit deuxième conduit est en communication de fluide avec ladite deuxième cavité.

13. Dispositif de cathéter à conduits multiples selon la revendication 12, comprenant de plus un conduit de fil de guidage, ledit conduit de fil de guidage s'étendant à partir de ladite première cavité à travers ladite extrémité dudit bout.

14. Dispositif de cathéter à conduits multiples selon la revendication 12, comprenant de plus un conduit de fil de guidage, ledit conduit de fil de guidage s'étendant à partir de ladite deuxième cavité à travers ladite extrémité dudit bout.

15. Dispositif de cathéter à conduits multiples selon la revendication 1, dans lequel une surface extérieure dudit cathéter intérieur est fixée audit cathéter extérieur au voisinage d'une extrémité distale dudit cathéter extérieur.

16. Dispositif de cathéter à conduits multiples selon la revendication 1, dans lequel ladite paroi extérieure comprend de plus une pluralité d'ouvertures disposées au voisinage d'une extrémité distale dudit cathéter extérieur, et dans lequel ladite région annulaire est en communication de fluide avec lesdites ouvertures.

17. Dispositif de cathéter à conduits multiples selon la revendication 1, dans lequel ladite paroi de confinement comprend de plus une pluralité d'ouvertures disposées au voisinage dudit bout dudit cathéter intérieur, lesdits premier et deuxième conduits étant en communication de fluide avec lesdites ouvertures.

18. Dispositif de cathéter à conduits multiples selon la revendication 1, dans lequel une section transversale de ladite paroi de confinement dudit cathéter intérieur est circulaire.

19. Dispositif de cathéter à conduits multiples selon la revendication 1, dans lequel une section transversale de ladite paroi de confinement dudit cathéter intérieur est ovale.

20. Dispositif de cathéter à conduits multiples selon la revendication 1, dans lequel ladite cloison traverse ledit cathéter intérieur, et dans lequel une surface de section transversale des deux parmi lesdits premier et deuxième conduits est en forme de D.

21. Dispositif de cathéter à conduits multiples selon la revendication 1, dans lequel ladite cloison est incurvée.
